Europäisches Patentamt
European Patent Office
Office européen des brevets

Veröffentlichungsnummer: **0 284 937 A2**

# EUROPÄISCHE PATENTANMELDUNG

Anmeldenummer: **88104454.9**

Anmeldetag: **21.03.88**

Int. Cl.4: **C07C 51/00**, C07C 57/30

Priorität: **30.03.87 DE 3710516**

Veröffentlichungstag der Anmeldung: **05.10.88 Patentblatt 88/40**

Benannte Vertragsstaaten: **AT BE CH DE ES FR GB GR IT LI LU NL SE**

Anmelder: **Henkel Kommanditgesellschaft auf Aktien**
**Postfach 1100 Henkelstrasse 67**
**D-4000 Düsseldorf-Holthausen(DE)**

Erfinder: **Krause, Horst-Jürgen, Dr.**
**Am Nettchesfeld 22**
**D-4000 Düsseldorf(DE)**

## Verfahren zur Herstellung von 2-Benzylfettsäuren.

Ein neues Verfahren zur Herstellung von 2-Benzylfettsäuren und deren Estern der Formel

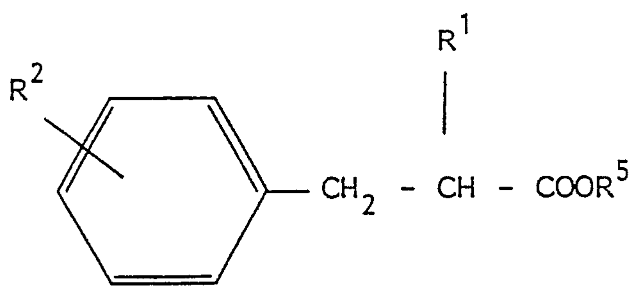

besteht darin, daß man 2-Fettalkyl-4,4-dimethyl-2-oxazoline mit Benzaldehyden der Formel $R^2$-$C_6H_4$-CHO zu 2-(1-Benzyliden)-fettalkyl-4,4-dimethyl-2-oxazolinen umsetzt, diese katalytisch zu den 2-(1-Benzyl)-fettalkyl-4,4-dimethyloxazolinen hydriert und diese durch säurekatalysierte Hydrolyse oder Solvolyse mit einem Alkohol der Formel $R^5$OH in die 2-Benzylfettsäuren oder deren Ester überführt. Als Katalysator für die Umsetzung der 2-Fettalkyl-4,4-dimethyl-2-oxazoline mit den Benzaldehyden liefert Amidosulfonsäure besonders hohe Ausbeuten.

EP 0 284 937 A2

Xerox Copy Centre

## "Verfahren zur Herstellung von 2-Benzylfettsäuren"

Die Erfindung betrifft ein neues Verfahren zur Gewinnung von 2-Benzylfettsäuren.

Modifizierte Fettsäuren, insbesondere gesättigte Fettsäuren mit einer Verzweigung an der ansonsten linearen Fettkette weisen gewisse anwendungstechnische Vorteile gegenüber den natürlichen, linearen Fettsäuren auf. Diese sind unter anderem dadurch bedingt, daß durch die Einführung einer Verzweigung in das Molekül das Molekulargewicht und der Siedepunkt erhöht und zugleich die Flüchtigkeit und der Schmelzpunkt gesenkt werden. Diese Vorteile sind nicht nur den Fettsäuren selbst, sondern auch den daraus herstellbaren Seifen, Estern, Amiden, Nitrilen, Aminen und deren Derivaten zueigen. Es hat daher nicht an Versuchen gefehlt, Substituenten in das Fettsäuremolekül einzubringen.

Es ist auch schon versucht worden, Benzylgruppen in die Alpha-Position (2-Position) von Fettsäuren einzuführen. V. H. Wallingford et al. (in J. Am. Chem. Soc. 64 (1942), 580 bis 582) haben versucht, durch Alkylierung von Malonestern, Hydrolyse und Decarboxylierung alphasubstituierte Fettsäuren, darunter auch 2-Benzyl-Stearinsäure herzustellen. Dieses Verfahren ist für eine technische Herstellung von 2-Benzylfettsäuren zu aufwendig.

H. L. Wehrmeister (in J. Org. Chem. 27 (1962), 4418 bis 4420) hat ein Verfahren zur Herstellung von kurzkettigen 2-Benzylidenalkansäuren angegeben, bei welchem 2-Alkyl-4,4-dimethyl-2-oxazoline mit Benzaldehyd kondensiert und das dabei erhaltene 2-2-(1-Benzyliden)-alkyl-4,4-dimethyl-2-oxazolin durch Hydrolyse in die 2-Benzylidenalkansäure überführt wird. Eine Übertragung dieses Verfahrens auf 2-Fettalkyl-4,4-dimethyl-2-oxazoline war unter Verwendung der in dieser Druckschrift angegebenen Katalysatoren für die Kondensation mit dem Benzaldehyd nicht oder nur in sehr geringen Ausbeuten von weniger als 40 % möglich.

Erst die Auffindung geeigneter Reaktionsbedingungen und eines für diese Reaktion geeigneten Katalysators machte es möglich, ein neues und für die Durchführung im technischen Maßstab geeignetes Verfahren zur Herstellung von 2-Benzylfettsäuren vorzuschlagen.

Gegenstand der Erfindung ist ein neues Verfahren zur Herstellung von 2-Benzylfettsäuren und deren Ester der Formel (I)

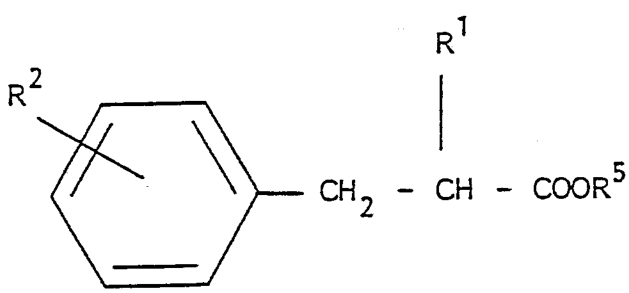

(I)

in der $R^1$ eine lineare Alkyl-oder Alkenylgruppe mit 4 bis 20 C-Atomen und $R^2$ = Wasserstoff, eine Alkylgruppe mit 1 bis 4 C-Atomen, Chlor, Brom, eine Nitrogruppe, eine Gruppe $-OR^3$, worin $R^3$ eine Alkylgruppe mit 1 bis 4 C-Atomen ist oder eine Gruppe $-NR^3R^4$ ist, worin $R^3$ und $R^4$ Alkylgruppen mit 1 bis 4 C-Atomen sind oder gemeinsam mit dem Stickstoffatom einen Pyrrolidin-, Piperidin-, Morpholin-oder Piperazinring bilden oder $R^3$ = Wasserstoff und $R^4$ eine Acylgruppe mit 1 bis 4 C-Atomen ist und $R^5$ Wasserstoff oder eine Alkylgruppe mit 1 bis 4 C-Atomen ist, dadurch gekennzeichnet, daß man 2-Fettalkyl-4,4-dimethyl-2-oxazoline der Formel (II)

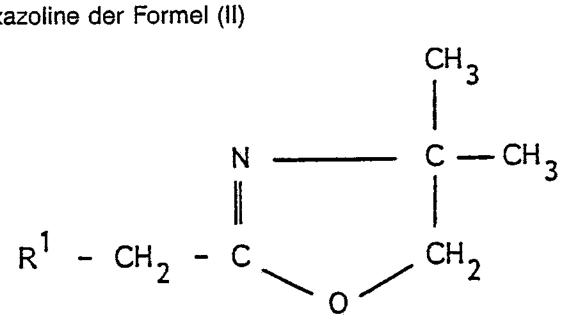

(II)

mit Benzaldehyden der Formel $R^2$-$C_6H_4$-CHO zu 2-(1-Benzyliden)-fettalkyl-4,4-dimethyl-2-oxazolinen der Formel (III)

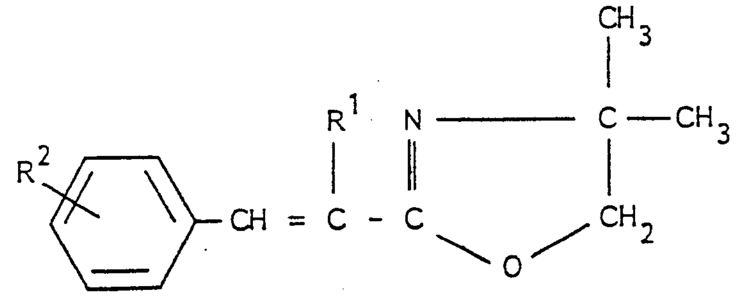

(III)

umsetzt, diese katalytisch zu 2-(1-Benzyl)-fettalkyl-4,4-dimethyloxazolinen hydriert und diese durch säurekatalysierte Hydrolyse oder Solvolyse mit einem Alkohol der Formel $R^5$OH in die 2-Benzylfettsäuren oder deren Ester der Formel (I) überführt.

In den Formeln (II) und (III) hat $R^1$ und $R^2$ die gleiche Bedeutung wie in Formel (I). Bevorzugt hat $R^2$ die Bedeutung Wasserstoff.

Die Herstellung der 2-Fettalkyl-4,4-dimethyl-2-oxazoline der Formel (II) ist bekannt. Ein geeignetes Verfahren zu ihrer Herstellung aus Fettsäuren mit 6 bis 18 C-Atomen und 2-Amino-2-methylpropanol wird z. B. von S. Serota et al. in J. Org. Chem. 46 (1981), 4147 bis 4151 angegeben.

Benzaldehyde der Formel $R^2$-$C_6H_4$CHO sind literaturbekannt und überwiegend im Handel erhältlich.

Die Umsetzung der 2-Fettalkyl-4,4-dimethyl-2-oxazoline der Formel (II) mit Benzaldehyden der Formel $R^2$-$C_6H_4$-CHO wird bevorzugt in Gegenwart von Amidosulfonsäure als Katalysator durchgeführt. Hierbei wird am besten bei einer Temperatur von 130 bis 160 °C in einem inerten Lösungsmittel gearbeitet, welches die azeotrope Entfernung des bei der Kondensation gebildeten Wassers gestattet. Man verwendet hierfür bevorzugt Alkylbenzole mit einem Siedepunkt oberhalb 130 °C, z. B. Ethylbenzol, Xylole oder Cumol. Bevorzugt geeignet ist Cumol. Bevorzugt wird das 2-Fettalkyl-4,4-dimethyl-2-oxazolin mit einem Teil (10 bis 30 %) des Benzaldehyds und mit dem Katalysator (ca. 0,1 Mol Amidosulfonsäure pro Mol Oxazolin) vorgelegt und der übrige Benzaldehyd (bis zu einer Menge von insgesamt ca. 1,1 Mol pro Mol Oxazolin) nach und nach während der Kondensation und azeotropen Entfernung des Wassers zugegeben.

Bei dieser Verfahrensweise wird eine Ausbeute von 85 bis 95 % (bezogen auf das Oxazolin) an 2-(1-Benzyliden)-fettalkyl-4,4-dimethyl-2-oxazolin erzielt, die eine Anwendung des Verfahrens in technischem Maßstab ermöglicht.

Die Hydrierung der 2-(1-Benzyliden)-fettalkyl-4,4-dimethyl-2-oxazoline wird nach einer an sich bekannten Verfahrensweise in einem inerten Lösungsmittel, z. B. in einem Alkylbenzol wie Toluol, Xylol, Ethylbenzol oder Cumol oder auch ohne Lösungsmittel in Gegenwart eines Hydrierkatalysators, z. B. eines Katalysators der Platin-Gruppe durchgeführt. Besonders geeignet ist Palladium auf einem Träger, z. B. Pd-Kohle mit ca. 5 Gew.-% Pd auf Aktivkohle. Von einem solchen Katalysator werden etwa 0,1 Gewichtsteil pro Gewichsteil des Oxazolins eingesetzt. Ein anderer besonders geeigneter Hydrierkatalysator ist hochaktives Nickel, z. B. in der Form von Raney-Nickel oder von handelsüblichem "Girdler Nickel G49B". Bei Verwendung von Pd-Kohle kann die Hydrierung bei Temperaturen von 70 bis 100 °C durchgeführt werden. Bei Verwen dung aktiver Nickelkatalysatoren ist eine etwas höhere Temperatur von 100 bis 130 °C erforderlich, um in vergleichbar kurzer Zeit eine vollständige Hydrierung der Doppelbindung zu erreichen. Die Hydrierung verläuft exotherm, so daß sich bei größeren Ansätzen eine Kühlung zur Einhaltung der optimalen Hydriertemperatur empfiehlt. Unter den genannten Bedingungen lassen sich Ausbeuten von 85 bis 95 % an 2-(1-Benzyl)-fettalkyl-4,4-dimethyl-2-oxazolinen erreichen.

Es hat sich als zweckmäßig erwiesen, die 2-(1-Benzyliden)-fettalkyl-4,4-dimethyl-2-oxazoline der Formel (II) ohne destillative Reinigung in die Hydrierung einzusetzen und erst nach der Hydrierung die 2-(1-Benzyl)-fettalkyl-4,4-dimethyl-2-oxazoline durch Destillation zu reinigen.

Die säurekatalysierte Hydrolyse der 2-(1-Benzyl)-fettalkyl-4,4-dimethyl-2-oxazoline wird durch Kochen mit wäßrigen Mineralsäuren, bevorzugt mit konzentrierter wäßriger Salzsäure durchgeführt. Dabei bildet sich wasserunlösliche 2-Benzylfettsäure und das wasserlösliche Salz des 2-Amino-2-methylpropanols. Die 2-Benzylfettsäuren lassen sich nach dem Abkühlen des Reaktionsgemisches leicht abtrennen und sind nach dem Waschen mit Wasser und Trocknen für die meisten Verwendungszwecke hinreichend rein. Erforderlichenfalls kann aber eine destillative Reinigung, oder bei den 2-Benzylfettsäuren auf Basis von Fettsäuren mit 14 und mehr Kohlenstoffatomen ($R^1$ = $C_{12}H_{25}$ bis $C_{20}H_{41}$) auch durch Umkristallisation durchgeführt werden.

Eine Möglichkeit zur direkten Herstellung von 2-Benzylfettsäureestern niederer Alkohole mit 1 bis 4 C-Atomen aus den 2-(1-Benzyl)-fettalkyl-4,4-dimethyl-2-oxazolinen besteht in einer Solvolyse in einem Alkohol $R^5OH$, worin $R^5$ eine Alkylgruppe mit 1 bis 4 C-Atomen ist, in Gegenwart einer Mineralsäure. So kann z. B. in Methanol/HCl eine direkte Solvolyse zu den 2-(1-Benzyl)-fettsäuremethylestern erreicht werden. Als saure Katalysatoren eignen sich alle hierfür bekannten Mineralsäuren und niedermolekularen Sulfosäuren, bevorzugt wird aber Salzsäure verwendet.

Aus der wäßrigen Phase des bei der Hydrolyse anfallenden Reaktionsgemisches kann durch Alkalisierung, z. B. mit einem Alkalihydroxid das 2-Amino-2-methyl-propanol freigesetzt und zurückgewonnen werden. Wenn man eine Aufarbeitung dieser wäßriger Phase und eine Rückführung des 2-Amino-2-methylpropanols anstrebt, was für eine großtechnische Herstellung unumgänglich ist, so sollte als Hydrolyse-Katalysator keine Schwefelsäure verwendet werden, da diese mit dem 2-Amino-2-methyl-propanol zu Aziridinen reagiert.

Die folgenden Beispiele sollen den Erfindungsgegenstand näher erläutern, ohne ihn hierauf zu beschränken.

## Beispiele

1. Herstellung von 2-Fettalkyl-4,4-dimethyl-2-oxazolinen der Formel (II) (allgemeine Vorschrift)

In einer Rührapparatur mit Destillationskolonne, Tropftrichter und Gaseinleitungsrohr wurde 2 Mol 2-Amino-2-methyl-1-propanol vorgelegt. Anschließend wurde 1 Mol Fettsäure langsam zugetropft (exotherme Reaktion).

Die Reaktionsmischung wurde dann unter Rühren und Stickstoff auf 120 bis 140 °C erhitzt, wobei das Reaktionswasser laufend azeotrop abdestilliert wurde, bis eine Brüdentemperatur von 105 °C erreicht war (Amid-Bildung).

Unter Steigerung der Reaktionstemperatur auf 180 °C wurde dann erneut Wasser abgespalten (Oxazolin-Bildung).

Das Erhitzen wurde solange fortgesetzt, bis eine Brüdentemperatur von 165 °C (reines Aminomethylpropanol (AMP)) erreicht war. Anschließend wurde überschüssiges AMP im Strahlervakuum abdestilliert und das so erhaltene 2-Fettalkyl-4,4-dimethyl-2-oxazolin fraktioniert destilliert.

Die Ausbeute an reinem 2-Fettalkyl-4,4-dimethyl-2-oxazolin lagen über 85 %.

Die Analysendaten der auf diese Weise erhaltenen 2-Fettalkyl-4,4-dimethyl-2-oxazoline der Formel (II) sind der Tabelle I zu entnehmen.

Tabelle I

| Nr. | Ausgangsfett-säure | $R^1$ | Siedepunkt | $n_D^{20}$ |
|---|---|---|---|---|
| 1.1 | Caprylsäure | 1-Hexyl | 113 °C (2 mbar) | 1.4401 |
| 1.2 | Caprinsäure | 1-Octyl | 140 °C (12 mbar) | 1.4442 |
| 1.3 | Laurinsäure | 1-Decyl | 112 °C (1 mbar) | 1.4498 |
| 1.4 | Palmitinsäure | 1-Tetradecyl | 141 °C (0,3 ") | 1.4522 |
| 1.5 | Stearinsäure | 1-Hexydecyl | 167 °C (0,001") | 1.4527 |

2. Herstellung von 2-(1-Benzyliden)-fettalkyl-4,4-dimethyl-2-oxazolinen der Formel (III) (allgemeine Vorschrift)

In einem Rührbehälter mit Wasserabscheider, Rückflußkühler und Tropftrichter wurden 1 Mol 2-Alkyl-4,4-dimethyl-2-oxazolin, 220 ml Cumol, 10 g Amidosulfonsäure und 0,2 Mol Benzaldehyd vorgelegt. Die Mischung wurde unter Rühren und Stickstoff bis auf Rückflußtemperatur (160 °C) aufgeheizt. Anschließend wurde innerhalb 1 Stunde 0,9 Mol Benzaldehyd zugetropft und das entstehende Reaktionswasser azeotrop abdestilliert. Nach Beendigung der Reaktion wurde abgekühlt, mit 50 ml wäßriger Natriumhydrogencarbonatlösung neutral gewaschen, die wäßrige Phase abgetrennt und die organische Phase destilliert.

Das Lösungsmittel und der überschüssige Benzaldehyd wurden im Wasserstrahlpumpenvakuum (15 Torr) abdestilliert.

Anschließend wurde das Reaktionsprodukt im Ölpumpenvakuum über einen Dünnschichtverdampfer destilliert.

Die Analysendaten der auf diese Weise erhaltenen Produkte der Formel (III) sind der Tabelle II zu entnehmen:

Tabelle II

| Nr. | Ausgangs-oxazolin | $R^1$ | $R^2$ | Siedepunkt (Dünnschichtverdampfer) | $n_D^{20}$ | Ausbeute |
|---|---|---|---|---|---|---|
| 2.1 | Beispiel 1 | 1-Hexyl | H | 130 °C (0,01 mbar) | 1.5330 | 90 % d.Th. |
| 2.2 | Beispiel 2 | 1-Octyl | H | 150 °C (0,01 mbar) | 1.5295 | 91 % d.Th. |
| 2.3 | Beispiel 3 | 1-Decyl | H | 170 °C (0,01 mbar) | 1.5223 | 85 % d.Th. |
| 2.4 | Beispiel 4 | 1-Tetradecyl | H | 180 °C (0,01 mbar) | 1.5135 | 91 % d.Th. |
| 2.5 | Beispiel 5 | 1-Hexadecyl | H | 200 °C (0,01 mbar) | | 88 % d.Th. |

3. Herstellung der 2-(1-Benzyl)-fettalkyl-4,4-dimethyl-2-oxazoline durch katalytische Hydrierung der 2-(1-Benzyliden)-fettalkyl-4,4-dimethyl-2-oxazoline (allgemeine Vorschrift)

In einem Hydrierautoklaven mit Rührer wurden die 2-(1-Benzyliden)-fettalkyl-4,4-dimethyl-2-oxazoline mit der gewichtsgleichen Menge an Cumol als Lösungsmittel (oder auch ohne Lösungsmittel) in Gegenwart von 10 Gew.-% eines Palladium auf Kohle-Katalysators (5 % Pd auf Kohle) bei 80 °C (oder bei 120 °C/Girdler-Nickel G49B) und 20 bar Wasserstoff hydriert.

Dabei wurden die Verbindungen der allgemeinen Formel (IV)

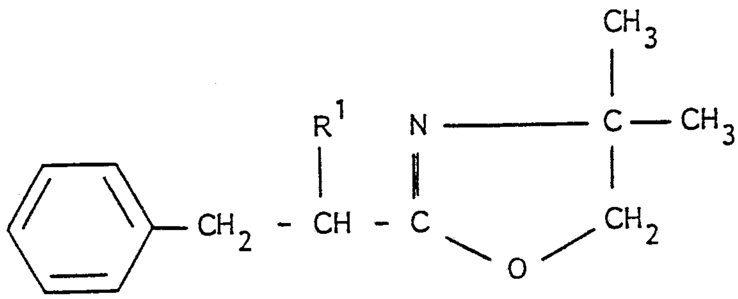

(IV)

mit den folgenden Analysendaten (Tabelle III) erhalten:

Tabelle III

| Nr. | Ausgangs-oxazolin | $R^1$ | Siedepunkt (Dünnschicht-verdampfer) | $n_D^{20}$ | Ausbeute |
|---|---|---|---|---|---|
| 3.1 | Beispiel 2.1 | 1-Hexyl | 130°C (0,01 mbar) | 1.4920 | 90 % |
| 3.2 | Beispiel 2.2 | 1-Octyl | 140°C (0,01 mbar) | 1.4876 | 93 % |
| 3.3 | Beispiel 2.3 | 1-Decyl | 170°C (0,01 mbar) | 1.4875 | 93 % |
| 3.4 | Beispiel 2.4 | 1-Tetradecyl | 180°C (0,01 mbar) | 1.4846 | 91,1% |
| 3.5 | Beispiel 2.5 | 1-Hexadecyl | 200°C (0,01 mbar) | 1.4840 | 85 % |

4. Herstellung der (±) 2-Benzyl-fettsäuren durch Hydrolyse der 2-(1-Benzyl)-fettalkyl-4,4-dimethyl-2-oxazoline (allgemeine Vorschrift)

In einer Rührapparatur mit Rückflußkühler und Gaseinleitungsrohr für Stickstoff wurde 1 Mol des jeweiligen 2-(1-Benzyl)-fettalkyl-4,4-dimethyl-2-oxazolins mit 500 ml konz. Salzsäure versetzt und unter Rühren und Stickstoff als Schutzgas 20 Stunden auf Rückflußtemperatur erhitzt.

Nach dem Abkühlen wurde die ölige Schicht (2-Benzyl-fettsäure) abgetrennt, mit Wasser gewaschen und im Ölpumpenvakuum über einen Dünnschichtverdampfer destilliert. Vielfach fielen die Säuren so rein an, daß eine Destillation nicht erforderlich war.

Ab $R^1$ = 1-Tetradecyl kristallisieren die Säuren nach dem Abkühlen bei 20 °C bereits aus. Diese werden abgesaugt und mit Wasser neutral gewaschen und gegebenenfalls umkristallisiert.

Die wäßrigen Lösungen, die das 2-Amino-2-methyl-1-propanol (AMP) Hydrochlorid enthalten, können zur Wiederverwendung des AMP aufgearbeitet werden.

Es wurden die in Tabelle IV aufgeführten Produkte der Formel (I) erhalten.

Tabelle IV

| Nr. | Ausgangs-oxazolin | $R^1$ = | Siedepunkt (Dünnschicht-verdampfer) / Schmelzpunkt | $n_D^{20}$ | Ausbeute |
|---|---|---|---|---|---|
| 4.1 | 3.1 | 1-Hexyl | 140°C (0,01 mbar) | 1.4975 | 97 % |
| 4.2 | 3.2 | 1-Octyl | 160°C (0,01 mbar) | 1.4949 | 98 % |
| 4.3 | 3.3 | 1-Decyl | 180°C (0,01 mbar) | 1.4923 | 99 % |
| 4.4 | 3.4 | 1-Tetradecyl | FP: 46°C | | 96 % |
| 4.5 | 3.5 | 1-Hexadecyl | FP: 55°C | | 98 % |

Die Struktur der Verbindungen wurde durch Kernresonanzspektrometrie (1H-NMR) bestätigt. Die Zusammensetzung aller in den Tabellen (I) bis (IV) aufgeführten Verbindungen wurde durch analytische Bestimmung des C-, H-und N-Gehaltes bestätigt.

**Ansprüche**

1. Verfahren zur Herstellung von 2-Benzylfettsäuren und deren Ester der Formel (I)

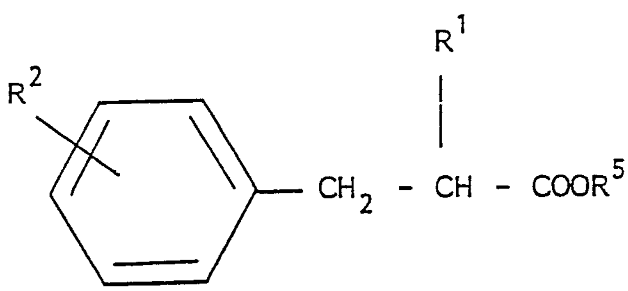

(I)

in der $R^1$ eine lineare Alkyl-oder Alkenylgruppe mit 4 bis 20 C-Atomen und $R^2$ = Wasserstoff, eine Alkylgruppe mit 1 bis 4 C-Atomen, Chlor, Brom, eine Nitrogruppe, eine Gruppe $-OR^3$, worin $R^3$ eine Alkylgruppe mit 1 bis 4 C-Atomen ist oder eine Gruppe $-NR^3R^4$ ist, worin $R^3$ und $R^4$ Alkylgruppen mit 1 bis 4 C-Atomen sind oder gemeinsam mit dem Stickstoffatom einen Pyrrolidin-, Piperidin-, Morpholin-oder Piperazinring bilden oder $R^3$ = Wasserstoff und $R^4$ eine Acylgruppe mit 1 bis 4 C-Atomen ist und $R^5$ Wasserstoff oder eine Alkylgruppe mit 1 bis 4 C-Atomen ist, dadurch gekennzeichnet, daß man 2-Fettalkyl-4,4-dimethyl-2-oxazoline der Formel (II)

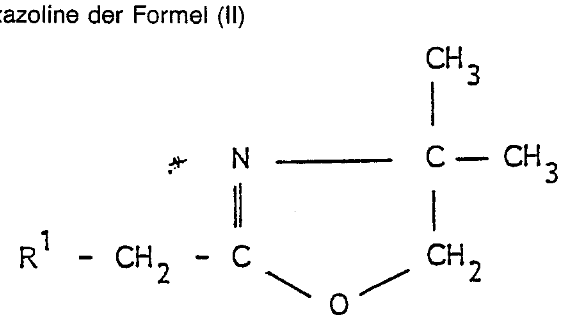

(II)

mit Benzaldehyden der Formel $R^2$-$C_6H_4$-CHO zu 2-(1-Benzyliden)-fettalkyl-4,4-dimethyl-2-oxazolinen der Formel (III)

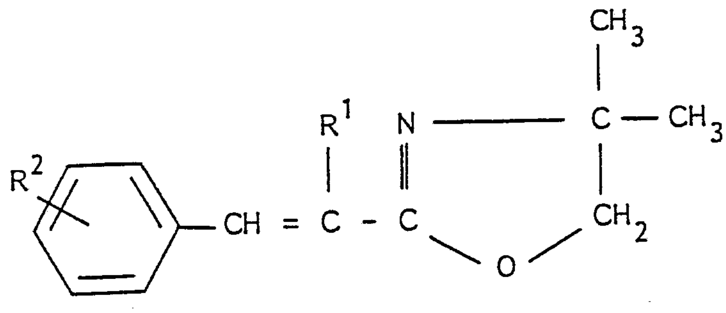

(III)

umsetzt, diese katalytisch zu 2-(1-Benzyl)-fettalkyl-4,4-dimethyloxazolinen hydriert und diese durch säurekatalysierte Hydrolyse oder Solvolyse mit einem Alkohol der Formel $R^5$OH in die 2-Benzylfettsäuren oder deren Ester der Formel (I) überführt.

2. Verfahren nach Anspruch 1, dadurch gekennzeichnet, daß die Umsetzung der 2-Fettalkyl-4,4-dimethyl-2-oxazoline der Formel (II) mit Benzaldehyden in Gegenwart von Amidosulfonsäure als Katalysator durchgeführt wird.

3. Verfahren nach den Ansprüchen 1 oder 2, dadurch gekennzeichnet, daß die Umsetzung der 2-Fettalkyl-4,4-dimethyl-2-oxazoline der Formel (II) mit Benzaldehyden bei einer Temperatur von 130 bis 160 °C in einem inerten, mit Wasser ein Azeotrop bildenden Lösungsmittel, bevorzugt in Cumol durchgeführt wird.

4. Verfahren nach den Ansprüchen 1 oder 2, dadurch gekennzeichnet, daß die Hydrierung des 2-(1-Benzyliden)-fettalkyl-4,4-dimethyl-2-oxazolin der Formel (III) in Gegenwart von Palladium-oder Nickelkatalysatoren durchgeführt wird.